# EUROPEAN PATENT APPLICATION

(11) **EP 4 670 783 A1**
(43) Date of publication of application: **31.12.2025**
(21) Application number: 25183937.9
(22) Date of filing: 19.06.2025
(51) Int. Cl.: A61N 5/10

(54) **SYSTEMS AND METHODS FOR GENERATING TREATMENT PLAN ALTERNATIVES FOR RADIATION THERAPY**

(30) Priority: 25.06.2024 US 202418753813
(71) Applicant: Siemens Healthineers International AG, 6312 Steinhausen (CH)
(72) Inventor: KUUSELA, Esa, 02320 Espoo (FI)
(74) Representative: Mathisen & Macara LLP

(57) **Abstract**

Provided herein are system (100) for generating radiation therapy treatment plan alternatives for radiation therapy. Systems (100) can include one or more processors to determine (202) a first treatment plan that satisfies an initial utility value, receive (204) data associated with a request to determine a second treatment plan from among the plurality of treatment plans, the request specifying a desired first metric value that is different from the first metric value of the first treatment plan; and determine (206) the second treatment plan from among the plurality of treatment plans, where the first metric value of the second treatment plan satisfies a first metric threshold when compared to first metric values of other treatment plans of the plurality of treatment plans. The one or more processors can provide (208) data associated with the second treatment plan to cause a device to operate in accordance with the second treatment plan.

## Description

### TECHNICAL FIELD

This application relates generally to systems and methods for generating radiation therapy treatment plan alternatives for radiation therapy and, in some non-limiting embodiments, to systems, methods, and non-transitory computer-readable mediums for generating treatment plan alternatives for radiation therapy based on reconstructed utility functions.

### BACKGROUND

Radiation therapy (referred to as radiotherapy (RT)) involves the delivery of radiation (energy) to targets within the body of a patient. For example, a multi-leaf collimator (MLC) coupled to a linear accelerator (LINAC) can be configured to move relative to a patient in accordance with a treatment plan to deliver energy to target tissue (e.g., tumors) at multiple control points. At each control point, the leaves of the MLC can be positioned and repositioned to form the shape of the beam generated by the LINAC. The goal of carefully planning the operation of the LINAC and shaping the beams using the MLC is to deliver radiation to the target tissue while minimizing the delivery of radiation to surrounding healthy tissue.

But conventional methods of optimizing treatment plans typically involve use of an automated treatment planner that abstracts certain aspects of the planning process. For example, a clinician such as an expert RT treatment planner can define parameters (sometimes referred to as a utility function) for a treatment plan and provide this utility function as an input to an automated treatment planner to generate an initial treatment plan candidate. Because the automated treatment planner abstracts aspects of the planning process and generates a single, optimized treatment plan, conventional techniques for developing treatment plans involves clinician iteratively reviewing the optimized treatment plans and updating the utility function to achieve certain target treatment objectives. These iterative changes are made until a desired treatment plan is generated. But iteratively changing the utility function can cause delay in the overall treatment plan generation process and unnecessarily consume computing resources used to generate and regenerate the treatment plans.

### SUMMARY

In accordance with a first aspect of the invention, there is provided a system as defined by claim 1.

In accordance with a second aspect of the invention, there is provided a method as defined by claim 8.

In accordance with a third aspect of the invention, there is provided a non-transitory computer-readable medium as defined by claim 14.

Optional features are defined by the dependent claims.

For the aforementioned reasons, there is a need for systems and methods that can generate alternative radiation treatment plans. Because conventional automated treatment planners provide as their output a single treatment plan that is optimized for one or more metrics based on a utility function (or cost function), these conventional automated treatment planners cannot generate alternative treatment plans that improve on one or more target metrics without reductions to other metrics that have a corresponding reduction on the overall utility (or increase in cost) when compared to the originally-generated treatment plan. To address this challenge, the techniques implemented by the systems and methods disclosed herein enable clinicians to update a treatment plan that is optimized by adjusting (improving or permitting a reduction) one or more metrics as opposed to the utility or cost function. For example, a clinician can initially configure a utility or cost function that is used by an automated treatment planner to identify a treatment plan that is optimized for a set of metrics used to evaluate a plurality of treatment plans. Upon review of the treatment plan, the clinician can provide input updating one or more of the metrics used to evaluate the plurality of treatment plans. In an example, the clinician can increase the metric corresponding to the dose delivered to a planning target volume while permitting a decrease in the overall utility (or increase in overall cost). The treatment planners described herein can then identify a different treatment plan that improves the metrics identified by the clinician while still and provide the different treatment plan as output for the clinician to review and/or to control a medical device as described herein.

In an embodiment, a system can comprise one or more processors that are programmed or configured to: determine a first treatment plan from among a plurality of treatment plans that satisfies an initial utility value. The initial utility value can be determined to indicate that the first treatment plan is optimized when compared to other treatment plans of the plurality of treatment plans based on a first utility function. A utility value of each treatment plan of the plurality of treatment plans can be based on (e.g., represented using) a first metric value associated with a first metric and a second metric value associated with a second metric for each treatment plan of the plurality of treatment plans. In some embodiments, the one or more processors may be programmed to receive data associated with a request to determine a second treatment plan from among the plurality of treatment plans, the request specifying a desired first metric value that is different from the first metric value of the first treatment plan. In some embodiments, the one or more processors may be programmed to determine the second treatment plan from among the plurality of treatment plans, where the first metric value of the second treatment plan satisfies a first metric threshold when compared to first metric values of other treatment plans of the plurality of treatment plans. In some embodiments, the one or more processors may be programmed to provide data associated with the second treatment plan to cause a device to operate in accordance with the second treatment plan.

In some embodiments, the desired first metric value may comprise an improvement in the first metric value. The request may further specify a desired utility value comprising an acceptable reduction in utility resulting from accommodating the improvement in the first metric value. A search space for the second treatment plan may be defined by the first metric value, the desired first metric value, the initial utility value, and the desired utility value. A treatment plan solution may be found in the search space wherein the first metric is as close as possible to the desired first metric value, and the utility is as close as possible to the desired utility value.

In some embodiments, the one or more processors that receive the data associated with the request can be programmed to receive the data associated with the request, the request specifying the desired first metric value and a desired utility value. The one or more processors that determine the second treatment plan from among the plurality of treatment plans can be programmed to determine the second treatment plan from among the plurality of treatment plans based on the first metric value of the second treatment plan satisfying the first metric threshold and the utility value of the second treatment plan satisfying a utility value threshold.

In at least some embodiments, the one or more processors can be programmed to determine the first treatment plan from among the plurality of treatment plans that satisfies the optimal utility value. In some embodiments, the one or more processors can be programmed to determine the first treatment plan from among the plurality of treatment plans based on a first utility function, the first utility function representing a first set of target metrics.

In some embodiments, the one or more processors be programmed to determine an updated first utility function based on the first utility function, the desired first metric value and the desired utility value specified by the request. The one or more processors that determine the second treatment plan from among the plurality of treatment plans can be programmed to: determine the second treatment plan based on an updated utility function, the updated utility function representing a second set of target metrics.

In embodiments, the first utility function can represent a first search space, and the updated utility function can represent a second search space that is at least in part different from the first search space. In some embodiments, the first search space can be bound by a first Pareto surface, and the second search space can be bound by a second Pareto surface. In some embodiments, the one or more processors that determine the second treatment plan from among the plurality of treatment plans can be programmed to: determine the second treatment plan from among the plurality of treatment plans, where the second treatment plan has a utility value that is lower than the utility value of the first treatment plan.

In some embodiments, the one or more processors may be programmed to receive data associated with a planning target volume (PTV) of a patient. The one or more processors that determine the first treatment plan from among the plurality of treatment plans that satisfies an optimal utility value can be programmed to: determine the first treatment plan from among the plurality of treatment plans, where the plurality of treatment plans represent operation of a linear accelerator (LINAC) delivering energy to the PTV of the patient.

In at least some embodiments, the first metric and the second metric can each represent one of: a target coverage of the PTV, a mean dose of energy delivered to the PTV, a maximum dose for an organ at risk (OAR), a mean dose of energy delivered to the OAR, or a complexity of a treatment plan.

In any or all of the embodiments, the first metric can represent one of: a target coverage of the PTV, a mean dose of energy delivered to the PTV, a maximum dose for an organ at risk (OAR), a mean dose of energy delivered to the OAR, or a complexity of a treatment plan. The second metric can represent one of: a target coverage of the PTV, a mean dose of energy delivered to the PTV, a maximum dose for an organ at risk (OAR), a mean dose of energy delivered to the OAR, or a complexity of a treatment plan. The first metric may represent a different metric from the metric represented by the second metric.

In another embodiment, a method includes determining, by at least one processor, a first treatment plan from among a plurality of treatment plans that satisfies an initial utility value. The initial utility value can be determined to indicate that the first treatment plan is optimized when compared to other treatment plans of the plurality of treatment plans based on a first utility function. A utility value of each treatment plan of the plurality of treatment plans can be based on (e.g., represented using) a first metric value associated with a first metric and a second metric value associated with a second metric for each treatment plan of the plurality of treatment plans. In some embodiments, the method can include receiving, by the at least one processor, data associated with a request to determine a second treatment plan from among the plurality of treatment plans, the request specifying a desired first metric value that is different from the first metric value of the first treatment plan. In some embodiments, the method can include determining, by the at least one processor, the second treatment plan from among the plurality of treatment plans, where the first metric value of the second treatment plan satisfies a first metric threshold when compared to first metric values of other treatment plans of the plurality of treatment plans. In some optional embodiments, the method can include providing data associated with the second treatment plan to cause a device to operate in accordance with the second treatment plan.

In some embodiments, the request can specify the desired first metric value and a desired utility value, and determining the second treatment plan from among the plurality of treatment plans can include determining the second treatment plan from among the plurality of treatment plans based on the first metric value of the second treatment plan satisfying the first metric threshold and the utility value of the second treatment plan satisfying a utility value threshold.

In at least some embodiments, determining the first treatment plan from among the plurality of treatment plans that satisfies the optimal utility value can include determining the first treatment plan from among the plurality of treatment plans based on a first utility function, the first utility function representing a first set of target metrics.

In embodiments, the method can include determining, by the at least one processor, an updated first utility function based on the first utility function, the desired first metric value and the desired utility value specified by the request. Determining the second treatment plan from among the plurality of treatment plans can include: determining, by the at least one processor, the second treatment plan based on an updated utility function, the updated utility function representing a second set of target metrics. In some embodiments, the first utility function represents a first search space, and wherein the updated utility function represents a second search space that is at least in part different from the first search space. The first search space can be bound by a first Pareto surface, and the second search space can be bound by a second Pareto surface.

In some embodiments, determining the second treatment plan from among the plurality of treatment plans can include determining the second treatment plan from among the plurality of treatment plans, where the second treatment plan has a utility value that is lower than the utility value of the first treatment plan.

In at least some embodiments, the method can include receiving, by the at least one processor, data associated with a planning target volume (PTV) of a patient. Determining the first treatment plan from among the plurality of treatment plans that satisfies an optimal utility value can include: determining the first treatment plan from among the plurality of treatment plans, where the plurality of treatment plans represent operation of a linear accelerator (LINAC) delivering energy to the PTV of the patient.

In embodiments, the first metric and the second metric each represent one of: a target coverage of the PTV, a mean dose of energy delivered to the PTV, a maximum dose for an organ at risk (OAR), a mean dose of energy delivered to the OAR, or a complexity of a treatment plan.

In an embodiment, a non-transitory computer-readable medium stores instructions thereon that, when executed by at least one processor, causes the at least one processor to: determine a first treatment plan from among a plurality of treatment plans that satisfies an initial utility value, the initial utility value determined to indicate that the first treatment plan is optimized when compared to other treatment plans of the plurality of treatment plans based on a first utility function, where a utility value of each treatment plan of the plurality of treatment plans is based on (e.g., represented using) a first metric value associated with a first metric and a second metric value associated with a second metric for each treatment plan of the plurality of treatment plans, receive data associated with a request to determine a second treatment plan from among the plurality of treatment plans, the request specifying a desired first metric value that is different from the first metric value of the first treatment plan; determine the second treatment plan from among the plurality of treatment plans, where the first metric value of the second treatment plan satisfies a first metric threshold when compared to first metric values of other treatment plans of the plurality of treatment plans; and provide data associated with the second treatment plan to cause a device to operate in accordance with the second treatment plan.

In some embodiments, the instructions that cause the at least one processor to receive the data associated with the request cause the at least one processor to receive the data associated with the request, the request specifying the desired first metric value and a desired utility value, and the instructions that cause the one or more processors to determine the second treatment plan from among the plurality of treatment plans cause the one or more processors to determine the second treatment plan from among the plurality of treatment plans based on the first metric value of the second treatment plan satisfying the first metric threshold and the utility value of the second treatment plan satisfying a utility value threshold.

In some embodiments, the instructions cause the at least one processor to determine the first treatment plan from among the plurality of treatment plans based on the first utility function, the first utility function representing a first set of target metrics.

In some embodiments, the instructions cause the at least one processor to determine an updated first utility function based on the first utility function, the desired first metric value and the desired utility value specified by the request, and determine the second treatment plan based on an updated utility function, the updated utility function representing a second set of target metrics.

In some embodiments, the instructions are configured so that the first utility function represents a first search space, and wherein the updated utility function represents a second search space that is at least in part different from the first search space.

In some embodiments, the instructions are configured so that the first search space is bound by a first Pareto surface, and wherein the second search space is bound by a second Pareto surface.

In some embodiments, the instructions are configured to determine the second treatment plan from among the plurality of treatment plans, where the second treatment plan has a utility value that is lower than the utility value of the first treatment plan.

In some embodiments, the instructions are configured to receive data associated with a planning target volume (PTV) of a patient and determine the first treatment plan from among the plurality of treatment plans, where the plurality of treatment plans represent operation of a linear accelerator (LINAC) delivering energy to the PTV of the patient.

In some embodiments, the instructions are configured so that the first metric and the second metric each represent one of: a target coverage of the PTV, a mean dose of energy delivered to the PTV, a maximum dose for an organ at risk (OAR), a mean dose of energy delivered to the OAR, or a complexity of a treatment plan.

By virtue of the implementation of the techniques described in associated with the above-noted systems and methods, the need for repetitive iteration when generating treatment plans can be reduced or eliminated. For example, clinicians engaging with systems and methods as described herein can define an initial utility function that causes a treatment planning system to generate an optimized treatment plan. Clinicians can then review the optimized treatment plan and provide updates to specific metrics represented by the utility function. The treatment planning system can then generate one or more updated treatment plans that are optimized based on both the original utility function, and the updates to the utility function. This results in fewer iterations by the clinician when interacting with the treatment planning system, which results in a similar reduction in computational resource consumption and faster convergence on a treatment plan that conforms to the target therapeutic goals of the clinician for the patient.

### BRIEF DESCRIPTION OF THE DRAWINGS

Non-limiting embodiments of the present disclosure are described by way of example with reference to the accompanying figures, which are schematic and are not intended to be drawn to scale. Unless indicated as representing the background art, the figures represent aspects of the disclosure.
**FIG. 1** illustrates a diagram of a system for generating radiation therapy treatment plan alternatives for radiation therapy, according to an embodiment.
**FIG. 2** illustrates a flow diagram of a process for generating radiation therapy treatment plan alternatives for radiation therapy, according to an embodiment.
**FIGS. 3A-3C** illustrate a flow diagram of an implementation involving generation of radiation therapy treatment plan alternatives for radiation therapy, according to an embodiment.
**FIGS. 4A-4C** illustrate diagrams of updated search spaces associated with updated metrics used to generate radiation therapy treatment pan alternatives, according to an embodiment.
**FIGS. 5A** and **5B** illustrate example functions, according to embodiments.

### DETAILED DESCRIPTION

Reference will now be made to the illustrative embodiments depicted in the drawings, and specific language will be used here to describe the same. It will nevertheless be understood that no limitation of the scope of the claims or this disclosure is thereby intended. Alterations and further modifications of the inventive features illustrated herein, and additional applications of the principles of the subject matter illustrated herein, which would occur to one skilled in the relevant art and having possession of this disclosure, are configured to be considered within the scope of the subject matter disclosed herein. Other embodiments can be used and/or other changes can be made without departing from the scope of the present disclosure. The illustrative embodiments described in the detailed description are not meant to be limiting of the subject matter presented.

The systems and methods described, as well as the techniques they implement, improve conventional RT treatment planning. More specifically, the systems and methods described enable a clinician to configure an initial utility or cost function for an automated treatment planner to identify a treatment plan that is optimized for a set of metrics used to evaluate a plurality of treatment plans. In embodiments, the systems can then receive input representing one or more target metrics involved in evaluating the plurality of treatment plans and then identify a different treatment plan that improves the metrics identified by the clinician. As a result, treatment plans can be quickly identified as optimal based on a given utility or cost function and one or more subsequent updates to the metrics involved in determining a given utility or cost, enabling a clinician to provide input that directly influences a given metric (e.g., increasing a dose delivered to a planning target volume, improving conformance to the planning target volume, and/or the like).

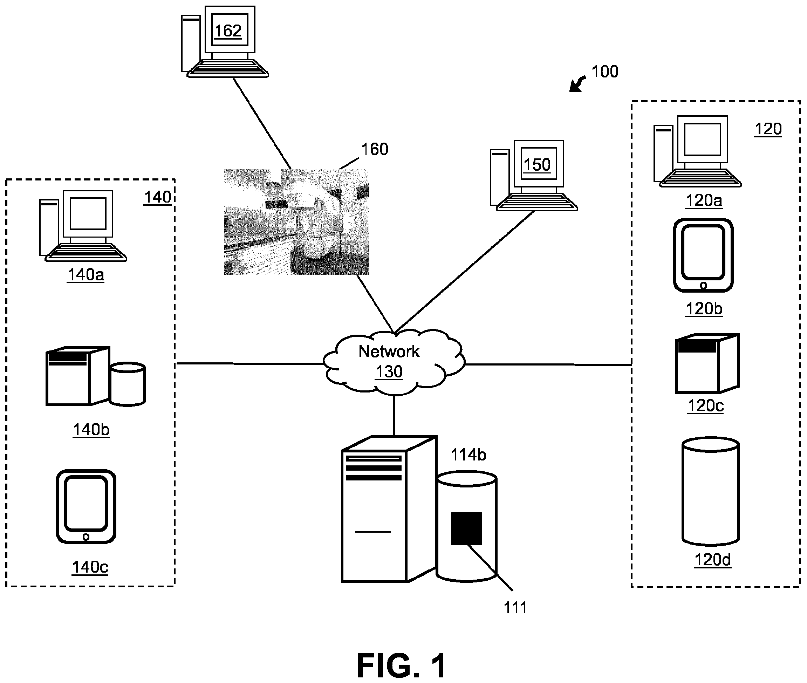

**FIG. 1** illustrates components of a system **100** for generating radiation therapy treatment plan alternatives for radiation therapy, according to an embodiment. The system **100** can include an analytics server **114a,** system database **114b,** a treatment planning system **111,** electronic data sources **120a-d** (each referred to individually as an electronic data source **120** and collectively electronic data sources **120,** unless stated otherwise), end-user devices **140a-c** (each referred to individually as an end user device **140** and collectively as end-user devices **140,** unless stated otherwise), an administrator computing device **150,** a medical device **160,** and medical device computer(s) **162.** Various components depicted in **FIG. 1** can belong to a radiotherapy clinic at which patients can receive radiotherapy treatment, in some cases via one or more radiotherapy machines located within the clinic (e.g., medical device **160**). The system **100** is not confined to the components described herein and can include additional or other components, not shown for brevity, which are configured to be considered within the scope of the embodiments described herein.

The above-mentioned components can be connected to each other through a network **130.** Examples of the network **130** can include, but are not limited to, private or public local-area-networks (LAN), wireless LAN (WLAN) networks, metropolitan area networks (MAN), wide-area networks (WAN), and the Internet. The network **130** can include wired and/or wireless communications according to one or more standards and/or via one or more transport mediums. The communication over the network **130** can be performed in accordance with various communication protocols such as Transmission Control Protocol and Internet Protocol (TCP/IP), User Datagram Protocol (UDP), and IEEE communication protocols. In one example, the network **130** can include wireless communications according to Bluetooth specification sets or another standard or proprietary wireless communication protocol. In another example, the network **130** can also include communications over a cellular network, including, e.g., a GSM (Global System for Mobile Communications), CDMA (Code Division Multiple Access), and EDGE (Enhanced Data for Global Evolution) network.

The analytics server **114a** can be any computing device comprising a processor and non-transitory machine-readable storage capable of executing the various tasks and processes described herein. The analytics server **114a** can employ various processors such as central processing units (CPU) and graphics processing unit (GPU), among others. Non-limiting examples of such computing devices can include workstation computers, laptop computers, server computers, and the like. While the system **100** includes a single analytics server **114a,** the analytics server **114a** can include any number of computing devices operating in a distributed computing environment, such as a cloud environment.

The analytics server **114a** can generate and display an electronic platform configured to use a treatment planning system **111** for receiving patient information, inputs from users (e.g., clinicians) such as utility functions and updated utility functions described herein, and outputting the results of execution of the treatment planning system **111.** The electronic platform can include graphical user interfaces (GUI) displayed by display devices of one or more electronic data sources **120,** the end-user devices **140,** the medical device **160,** and/or the administrator computing device **150.** An example of the electronic platform generated and hosted by the analytics server **114a** can be a web-based application or a website configured to be displayed on different electronic devices, such as mobile devices, tablets, personal computers, and the like.

The information displayed by the electronic platform can include, for example, input elements to receive data associated with a patient being treated, synchronize one or more sensors, and display results of predictions produced by the treatment planning system **111.** For instance, the analytics server **114a** can execute the treatment planning system **111** (e.g., a system that is configured and/or trained to generate fluence maps, leaf sequences, etc., as described herein for a patient being treated via the medical device **160**). The analytics server **114a** can then display the results for a clinician and/or directly revise one or more operational attributes of the medical device **160.**

The electronic data sources **120** can be any computing device comprising a processor and non-transitory machine-readable storage capable of executing the various tasks and processes described herein. For example, the electronic data sources **120** can represent various computing devices that contain, retrieve, and/or access data associated with a medical device **160,** such as data associated with operational information of currently or previously performed radiotherapy treatments (e.g., electronic log files or electronic configuration files), data associated with current and/or previously monitored patients (e.g., computed tomography (CT) scans, magnetic resonance imaging (MRI) scans, tumor locations, deformation information, and/or the like) or participants in a study, and/or the like. For instance, the analytics server **114a** can use the clinic computer **120a,** medical professional device **120b,** server **120c** (associated with a clinician and/or a clinic), and database **120d** (associated with the clinician and/or the clinic) to retrieve/receive data associated with the medical device **160.** The analytics server **114a** can retrieve the data from the end-user devices **120,** generate a dataset, and use the dataset to configure the treatment planning system **111** (e.g., models implemented by the treatment planning system **111** and/or the like). The analytics server **114a** can execute various algorithms to translate raw data received/retrieved from the electronic data sources **120** into machine-readable objects that can be stored and processed by other analytical processes as described herein.

End-user devices **140** can be any computing device comprising a processor and a non-transitory machine-readable storage medium capable of performing the various tasks and processes described herein. Non-limiting examples of an end-user device **140** can be a workstation computer, laptop computer, tablet computer, or server computer. In operation, various users such as clinicians as described herein can use end-user devices **140** to access the GUI operationally managed by the analytics server **114a** or otherwise the results of the execution of the treatment planning system **111.** Specifically, the end-user devices **140** can include clinic computer **140a,** clinic server **140b,** and a medical professional device **140c.** Even though referred to herein as "end-user" devices, these devices can not always be operated by end-users. For instance, the clinic server **140b** can not be directly used by an end user. However, the results stored on the clinic server **140b** can be used to populate various GUIs accessed by an end user via the medical professional device **140c.** In some embodiments, the end-user device **140** can be associated with one or more clinicians that are associated with the generation of one or more treatment plans (e.g., involved in preparing the one or more treatment plans) for patients.

The administrator computing device **150** can represent a computing device operated by a system administrator. The administrator computing device **150** can be configured to display radiotherapy treatment attributes generated by the analytics server **114a** (e.g., various analytic metrics determined during training of one or more machine learning models and/or systems); monitor various treatment planning systems **111** utilized by the analytics server **114a,** electronic data sources **120,** and/or end-user devices **140;** review feedback; and/or facilitate training or retraining (calibration) of the treatment planning system **111** that are maintained by the analytics server **114a.**

In some embodiments, the medical device **160** can be a diagnostic imaging device or a treatment delivery device. For example, the medical device **160** can include one or more computed tomography (CT) scanners, linear accelerators (LINACs) having a multi-leaf collimator (MLC) that consists of multiple small lead leaves that can be individually moved to shape the radiation beam and deliver the dose to the tumor while minimizing the dose to surrounding healthy tissues, or other similar devices configured to transmit energy toward targeted tissue (referred to as planning target volumes) associated with a patient and, in some cases, measure the energy transferred to ward the targeted tissue. The medical device **160** can also include one or more sensors configured to monitor the patient being treated. That is, the medical device **160** and/or the analytics server **114a** can be communicating with various sensors that can monitor a patient's external biological signals. Non-limiting examples of the sensors can include 3D surfacing mechanisms and optical (or other) sensors configured to monitor the patient's movements (e.g., how the patient is moving and/or breathing). In some embodiments, the medical device **160** can receive data associated with a treatment plan from the medical device computer(s) **162** that cause the medical device **160** to operate in accordance with the treatment plan.

The treatment planning system **111** can be stored in the system database **114b.** The treatment planning system **111** can be trained using data received/retrieved from the electronic data sources **120** and can be executed using data received from the end-user devices, the medical device **160,** and/or the sensor **163.** In some embodiments, the treatment planning system **111** can reside within a data repository local or specific to a clinic. In various embodiments, the treatment planning system **111** can use one or more deep learning engines to develop a treatment plan for a patient using radiation therapy. For instance, the analytics server **114a** can transmit patient attributes from the sensor **163** and execute the treatment planning system **111** accordingly. The analytics server **114a** can then display the results on one or more end-user devices **140.** In some embodiments, the analytics server **114a** can change one or more configurations of the medical device **160** based on the results predicted by the treatment planning system **111.**

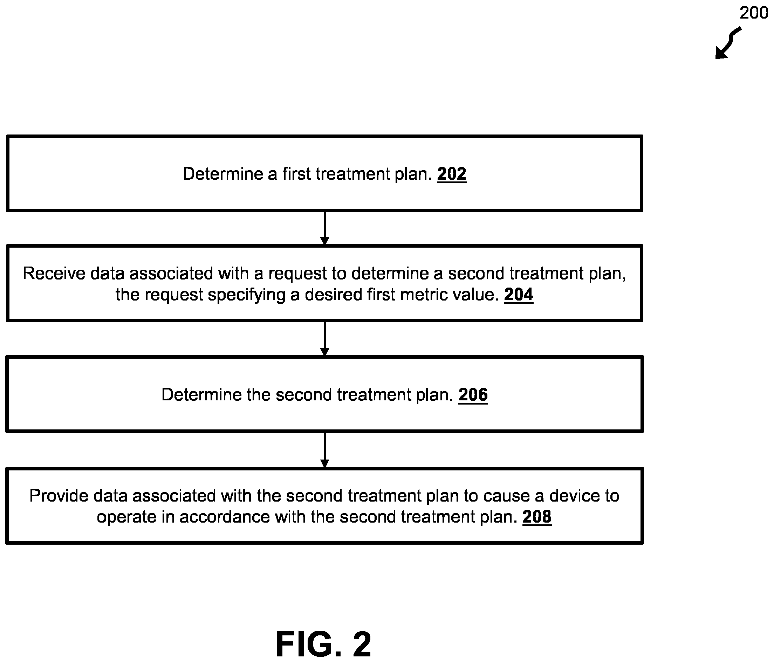

Referring to **FIG. 2****,** illustrated is a flow diagram of a process 200 for generating radiation therapy treatment plan alternatives for radiation therapy. The process **200** includes operations **202-208.** However, other embodiments can include additional or alternative operations or can omit one or more operations altogether. The process **200** is described as being executed by an analytics server, which can be the same as, or similar to, the analytics server **114a** described in **FIG. 1****.** However, one or more steps of the process **200** can be executed by any number of computing devices operating in the distributed computing system described in **FIG. 1****.** For instance, one or more computing devices can locally perform part or all of the steps described in **FIG. 2****.**

At operation **202,** the analytics server can determine a first treatment plan. For example, the analytics server can determine the first treatment plan based on the analytics server receiving data associated with a patient. The data associated with the patient can represent one or more of: one or more two-dimensional and/or three-dimensional scans (e.g., CT scans, MRI scans, and/or the like) of at least a portion of the patient that includes tissue that is being targeted to receive radiation (referred to herein as a planning target volume (PTV)). In these examples, the PTV can include cancerous tissue and/or the like.

In examples, the analytics server can determine the first treatment plan based on the analytics server providing the data associated with the patient to a treatment planning system. In some embodiments, the analytics server can cause the treatment planning system to generate an output. The output of the treatment planning system can be associated with (e.g., represent) a treatment plan. For example, the output of the treatment planning system can include a treatment plan for any combination of one or more of; (i) controlling operation of a LINAC during delivering of energy to the PTV of the patient, (ii) a sequence of control points to move the LINAC when delivering energy to the PTV of the patient, (iii) leaf positions and/or leaf motions of the MLC involved in forming beams generated by the LINAC, and (iv) energy doses (referred to as monitor units (MUs) usable to control the LINAC when delivering energy to the PTV of the patient, and/or the like.

In some embodiments, the analytics server determines the first treatment plan from among a plurality of possible treatment plans. For example, the analytics server can determine a treatment plan based on the analytics server iterating through one or more possible beam configurations and corresponding MUs across multiple possible treatment plans (candidate treatment plans) to determine a plan that optimizes a dose delivered to the PTV while minimizing the dose delivered to non-target tissue (sometimes referred to as organs at risk (OAR)). In examples, the analytics server can determine a utility value for one or more of the candidate treatment plans. In an example, the analytics server can determine values for one or more metrics usable to evaluate treatment plans and the analytics server can determine the utility value for each of the candidate treatment plans based on the value of the metrics corresponding to each candidate treatment plan. In some embodiments, the metrics can include any combination of one or more of: a target coverage of the PTV (indicating whether the PTV receives a dose, whether the PTV receives a specified dose, a degree to which the PTV does not receive the specified dose, and/or the like), a dose delivered to one or more PTVs and/or OARs (e.g., a dose as compared to a permitted dose, an average dose, and/or maximum dose), a treatment complexity, a treatment time, and/or the like.

In some embodiments, the analytics server determines that the first treatment plan satisfies an initial (e.g., optimal) utility value based on the analytics server determining utility values for the candidate treatment plans. For example, the analytics server can determine utility values for each of the candidate treatment plans based on the values for the one or more metrics corresponding to each of the candidate treatment plans. In this example, the analytics server can compare the utility values for each of the candidate treatment plans to the utility values of each of the other candidate treatment plans to determine that the first treatment plan satisfies the optimal utility value. In examples, the optimal utility value can be the highest utility value from among the utility values of each of the candidate treatment plans. While some embodiments herein are described with reference to a first metric value associated with a first metric and a second metric value associated with a second metric, it will be understood that the candidate treatment plans can be evaluated in accordance with any number of metrics.

In some embodiments, the analytics server determines the first treatment plan from among the plurality of candidate treatment plans based on a first utility function. For example, the analytics server can receive data associated with input provided by a user (e.g., a clinician) operating one or more of the devices of FIG. 1 such as the end user devices. The input provided by the user can be associated with one or more target metrics such as metric values or ranges of metric values (e.g., a first set of target metrics and/or ranges of target metrics) that can be used to represent a utility function. In one illustrative example, the input provided by the user can be associated with a first metric value (e.g., representing a target dose to be delivered to a PTV) and a second metric value (e.g., representing a three-dimensional region associated with (e.g., bounding) the PTV). In this illustrative example, the first metric value and second metric value can be combined to form the first utility function, where the first metric value and the second metric value represent metric values that are desired by the user. As discussed herein, the term target metric can indicate a desired metric specified by a user that may or may not be achieved by generated treatment plans. While the first utility function is described as being determinable based on one or more metric values, it will be understood that a utility function can be determined using one or more aspects of operation of a LINAC such as, for example, machine control points or dose distributions involved in delivering energy to a PTV of a patient.

In some embodiments, the first utility function can be associated with a first search space. For example, the first utility function can be a function that is configured to generate outputs that are within the first search space. In examples, where the treatment planning system is generating candidate treatment plans, the treatment plans can be evaluated using the first utility function to generate corresponding utility values representing a first search space. In some examples, the first utility function can generate multiple candidate treatment plans having similar utility values bound within the first search space (e.g., by a Pareto surface).

At operation **204,** the analytics server can receive data associated with a request to determine a second treatment plan. For example, the analytics server can receive the data associated with the request to determine the second treatment plan from among a plurality of treatment plans. In examples, the plurality of treatment plans can include a subset of the candidate treatment plans. In some embodiments, the request specifies a desired first metric value that is different from the first metric value. For example, the analytics server can receive data associated with the request, where the request includes an updated input provided by a user, e.g. the user that provided the input representing the first utility function. The input provided by the user can be associated with the one or more target metrics such as metric values or ranges of metric values that represent updates to the first utility function (e.g., a second set of target metrics and/or ranges of target metrics) and are different from corresponding metric values associated with the treatment plan generated based on the first utility function. In one illustrative example, the input provided by the user can be associated with an updated first metric value (e.g., an increase to a value representing a target dose to be delivered to a PTV). In this illustrative example, the analytics server can update the first metric value of the first utility function based on the updated first metric value to determine an updated first utility function.

In some embodiments, the request can also specify a desired utility value. For example, the request can include a desired utility value that is lower than the utility value of the first treatment plan. As an illustrative example, a user can first review the first treatment plan and determine that it would be desirable to increase the dose delivered to the PTV of the patient. In this illustrative example, the user can also specify a desired utility value that corresponds to an acceptable reduction in the utility value of the first treatment plan. In this way, because the first treatment plan is optimized and is associated with the highest possible utility value given the first utility function, the user can specify an improvement to a given metric (e.g., an increased dose delivered to the PTV of the patient) while also specifying an acceptable offset in overall utility (e.g., resulting in an increase in dose delivered to one or more OARs). That is, the user can specify an improvement to a given metric whilst also specifying an acceptable reduction in overall utility resulting from improving the given metric.

In some embodiments, the analytics server can determine an updated utility function (e.g., an updated first utility function). For example, the analytics server can determine an updated utility function based on the first utility function, the desired first metric value, and the desired utility value. In an example, the analytics server can determine the updated utility function based on the analytics server replacing one or more of the metric values of the first utility function (e.g., of the first set of target metrics) with the corresponding metric values specified by the request (e.g., a second set of target metrics). In some embodiments, the analytics server can determine the updated utility function, where the updated utility function is associated with a desired utility value. For example, where the request specifies a desired utility value, the updated utility function can be associated with the desired utility value.

In some embodiments, the updated first utility function can be associated with a second search space. For example, the updated first utility function can be a function that is configured to generate outputs that are within both the first search space and the second search space. One or more generated outputs within the first search space may not be within the second search space, and one or more generated outputs within the second search space may not be within the first search space. In examples, where the treatment planning system is generating candidate treatment plans (e.g., candidate treatment plans that are associated with metric values and utility values that satisfy the updated metric value and the updated utility value), the treatment plans can be evaluated using the updated first utility function to generate corresponding utility values within a second search space. In some examples, the updated first utility function can generate multiple candidate treatment plans having similar utility values bound within the second search space (e.g., by a second Pareto surface). In some embodiments, the updated utility function can also be updated to indicate one or more metrics that are configured to be disregarded when calculating utility values for candidate treatment plans.

At operation **206,** the analytics server can determine the second treatment plan. For example, the analytics server can determine a second treatment plan based on the analytics server receiving the data associated with the request to determine the second treatment plan. In examples, the analytics server can determine the second treatment plan based on the analytics server providing the data associated with the patient to a treatment planning system similar to as described above. In some embodiments, the analytics server can cause the treatment planning system to generate an output. The output of the treatment planning system can be associated with a treatment plan as described herein.

In some embodiments, the analytics server determines the second treatment plan from among a plurality of possible treatment plans. For example, the analytics server can determine a treatment plan based on the analytics server iterating through one or more possible beam configurations and corresponding MUs across multiple possible treatment plans to determine a plan that optimizes a dose delivered to the PTV while minimizing the dose delivered to OARs. In examples, the analytics server can determine a utility value for one or more of the candidate treatment plans based on the utility functions as described herein. For example, the analytics server can determine the utility value for the one or more candidate treatment plans based on the updated first utility function described herein. For example, the analytics server can determine the utility value of each of the candidate treatment plans (e.g., the candidate treatment plans generated prior to, or after the analytics server receives the request to determine the second treatment plan) in accordance with the updated first utility function. The analytics server can then determine the second treatment plan from among the plurality of candidate treatment plans based on a utility value of the second treatment plan satisfying a utility value threshold. In this example, the utility value threshold can be less than the utility value threshold associated with the first treatment plan.

In examples, the analytics server can filter one or more of the candidate treatment plans based on whether each of the candidate treatment plans satisfies the one or more updated metrics specified by the request to generate the second treatment plan. For example, where a given candidate treatment plan is associated with a metric value for a given metric that does not satisfy the one or more metric values (or ranges of metric values) specified by the request to determine the second treatment plan, the analytics server can filter (e.g., remove) the given candidate treatment plan. The analytics server can then determine the second treatment plan based on the second treatment plan satisfying the utility value threshold (e.g., the utility value threshold specified by the request to determine the second treatment plan). As described herein, the utility value of the second treatment plan can be less than the utility value of the first treatment plan, enabling the second treatment plan to be considered where a given metric or set of metrics are improved at the cost of other metrics.

At optional operation **208,** the analytics server can provide data associated with the second treatment plan to cause a device to operate in accordance with the second treatment plan. For example, the analytics server can provide the data associated with the second treatment plan to a medical computing device (e.g., a medical computing device that is the same as, or similar to, the medical computing device of FIG. 1) to cause a medical device (e.g., a medical device that is the same as, or similar to, the medical device 160 of FIG. 1) to operate in accordance with the second treatment plan.

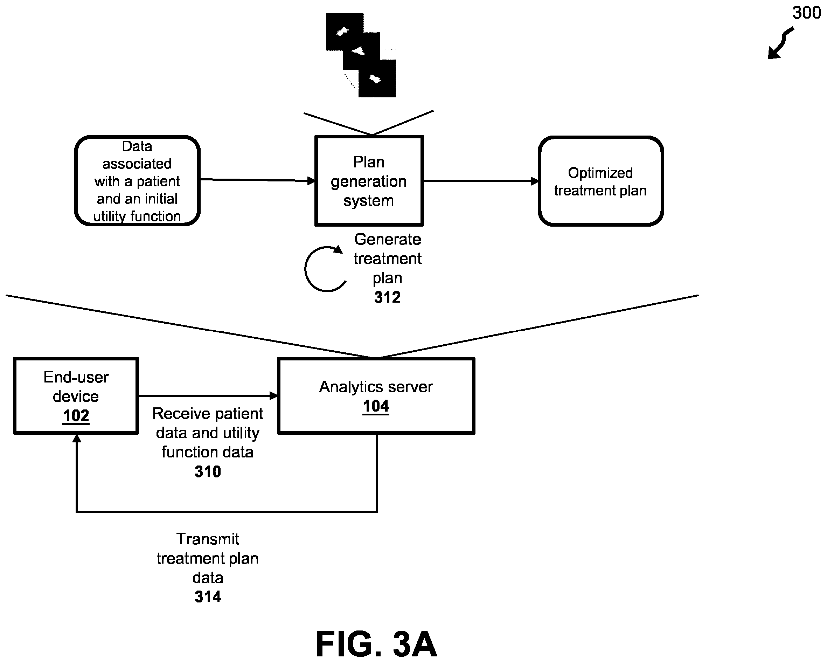

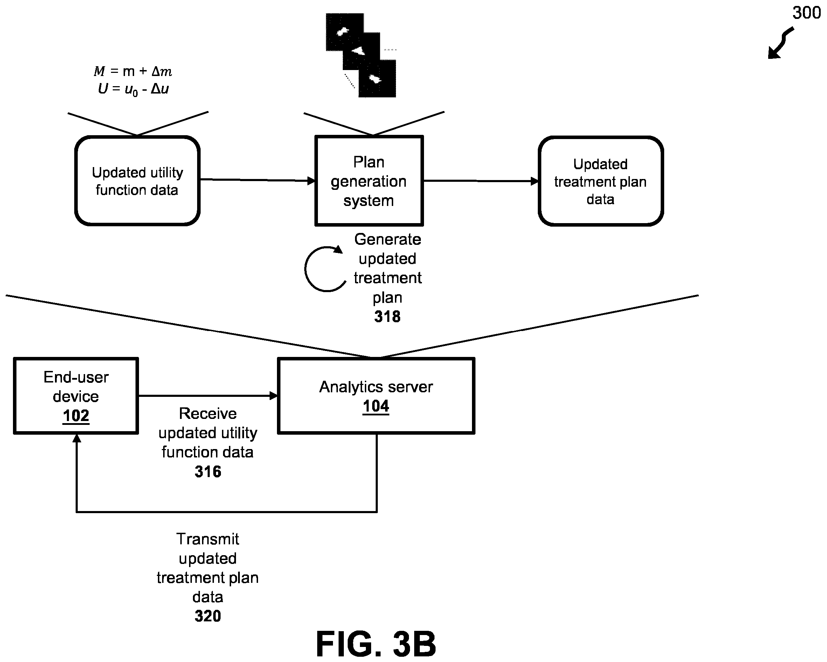

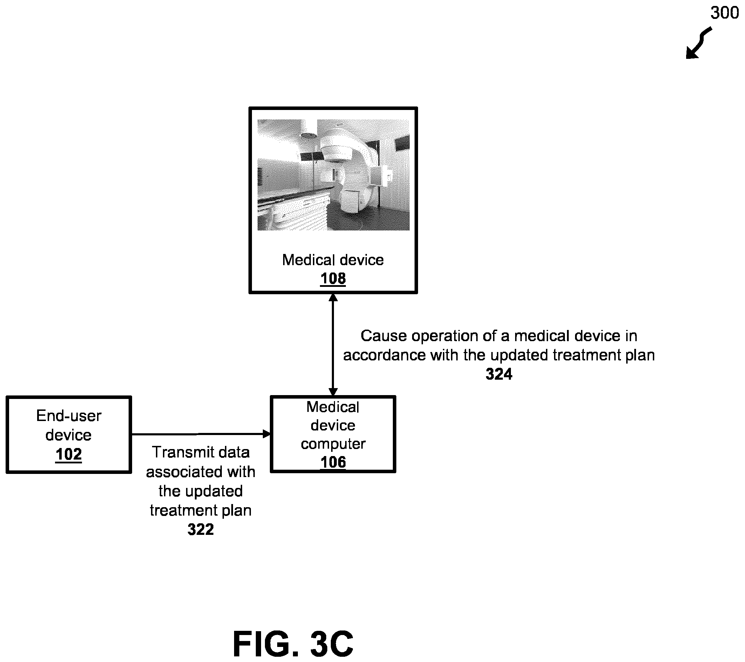

Referring now to **FIGS. 3A-3C****,** illustrated is an example flow diagram of an implementation **300** involving generation of radiation therapy treatment plan alternatives for radiation therapy, according to an embodiment. As illustrated in **FIGS. 3A-3C****,** the implementation **300** involves an end-user device **102,** an analytics server **104,** a medical device computer **106,** and a medical device **108.** In some embodiments, the end-user device **102** can be the same as, or similar to, the end user devices **140** of **FIG. 1****;** the analytics server **104** can be the same as, or similar to, the analytics server **114a** of **FIG. 1****;** the medical device computer **106** can be the same as, or similar to, the medical device computer **162** of **FIG. 1****;** and/or the medical device **108** can be the same as, or similar to, the medical device **160** of **FIG. 1****.**

At step **310,** the analytics server **104** receives patient data and utility function data from an end-user device **102.** For example, the analytics server **104** can receive the patient data where the patient data is associated with a patient and the utility function data is associated with a first set of target metrics.

At step **312,** the analytics server **104** can generate a treatment plan. The analytics server **104** can provide the patient data and/or the utility function data to a plan generation system to cause the plan generation system to generate an initial (e.g., optimal) treatment plan. For example, the analytics server **104** can iterate through one or more possible beam configurations and corresponding MUs across multiple possible treatment plans (candidate treatment plans) to determine a plan that optimizes a dose delivered to the PTV while minimizing the dose delivered to OARs.

At step **314,** the analytics server **104** transmits treatment plan data associated with the treatment plan (generated at step **312**) to the end-user device **102.** For example, the analytics server **104** can transmit the treatment plan data associated with the treatment plan to the end-user device **102,** where the treatment plan data is configured to cause a display device of the end-user device **102** (not explicitly illustrated) to display a representation of the treatment plan. In some embodiments, a clinician operating the end-user device **102** can provide input via the end-user device **102** causing the end-user device **102** to generate a request to generate a second treatment plan. The request can specify one or more updated metric values (e.g., = m + \delta m; or e.g., = m + Δm) and/or one or more updated utility values (e.g., *U* = *u*₀ - Δu).

At step **316,** the analytics server **104** receives updated utility function data. For example, the analytics server **104** receives updated utility function data from the end-user device **102** based on the clinician providing the input representing the request to generate the second treatment plan to the end-user device **102.**

At step **318,** the analytics server **104** generates an updated treatment plan. For example, the analytics server **104** can generate the updated treatment plan by providing the updated metric values and/or the updated utility values to the plan generation system (sometimes referred to herein as a treatment planning system). In examples, the plan generation system can generate the updated treatment plan by generating one or more candidate treatment plans based on (e.g., in accordance with) the updated utility function data.

At step **320,** the analytics server **104** transmits treatment plan data associated with the updated treatment plan (generated at step **318**) to the end-user device **102.** For example, the analytics server **104** can transmit the treatment plan data associated with the updated treatment plan to the end-user device **102,** where the treatment plan data is configured to cause a display device of the end-user device **102** to display a representation of the updated treatment plan. In some embodiments, a clinician operating the end-user device **102** can again provide input via the end-user device **102** causing the end-user device **102** to generate a request to generate another second treatment plan. In examples, the clinician can provide input to the end-user device **102** to cause the updated treatment plan to be accepted.

At step **322,** the end-user device **102** transmits data associated with the updated treatment plan to a medical device computer **106.** For example, the end-user device **102** can transmit the data associated with the updated treatment plan to the medical device computer **106** based on the end-user device **102** receiving input indicating the updated treatment plan is accepted.

At optional step **324,** the medical device computer **106** can cause operation of a medical device **108** in accordance with the updated treatment plan. For example, the medical device computer **106** can cause operation of a medical device **108** in accordance with the updated treatment plan based on the medical device computer **106** receiving the data associated with the updated treatment plan.

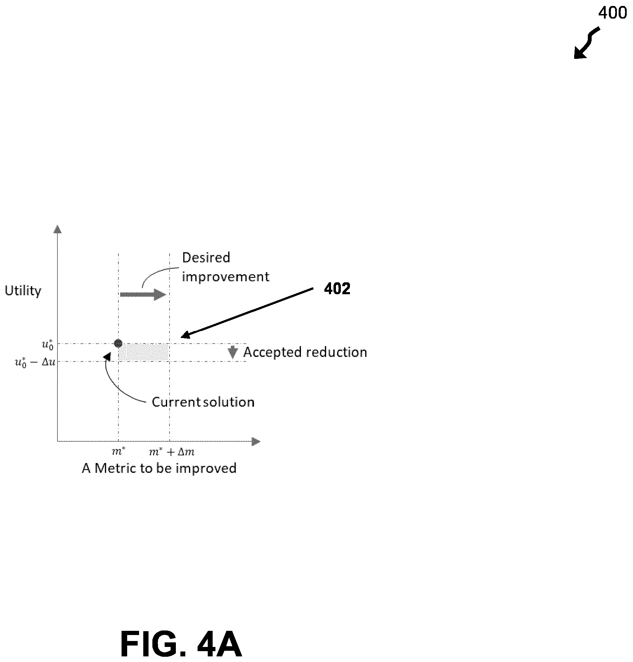

Referring now to **FIGS. 4A-4C****,** illustrated are example diagrams **400, 410, 420** of updated search spaces associated with updated metrics used to generate radiation therapy treatment plan alternatives.

As shown in **FIG. 4A****,** the diagram **400** metric values associated with a treatment plan (e.g., a first treatment plan as described herein) can be represented in accordance with a "current solution." Assuming that the plan generation system found an optimal treatment plan as a current solution, individual metrics can be improved only if a certain reduction is accepted for the general plan quality (as measured by the utility values generated in accordance with an original utility function). When setting the desired improvement of the selected metric and the accepted reduction to the original utility, the clinician is stating (e.g., defining) a new optimization problem.

The diagram **400** can include a point at *m *, u₀** representing a given metric value and utility value. In some embodiments, *u₀** can represent a utility value that is optimized (e.g., maximized) for a given range of metric values, and *m* * can represent a value of a metric associated with the treatment plan that corresponds to the optimized utility value generated using the original utility function. A spectrum of metric values can be represented along the x-axis, and a spectrum of utility values can be represented along the y-axis of the diagram **400.** In some embodiments, given the current solution, a clinician involved in developing a treatment plan for a patient targeting a PTV of the patient can specify an amount by which to increase the value of a given metric *M* along with an amount by which a utility *U* of a second treatment plan can be decreased to accommodate the increase of the value of the metric *M.* Because the current solution presents the current solution for the original utility function *U* having a utility of *u₀*,* the clinician may determine (e.g., observe) that the metrics represented along the x-axis is not quite meeting the clinician's expectations and wants to explore if an improvement to the metric with a certain overall reduction in utility for a second treatment plan can be generated. Since the current solution is maximizing the original utility, it is not feasible to expect that the selected metric could be improved without reduction in the utility. A new optimization task is defined by specifying an amount by which to increase the value of a given metric*M* along with an amount by which a utility *U* of a second treatment plan can be reduced as represented by a rectangular region **402** where the new solution (e.g., a second treatment plan) is searched. By updating the metric value and utility value, a plan generation system can determine a solution that is within this rectangle, that is as much in the right as possible - and secondarily as high in to top, or as high towards the top, as possible.

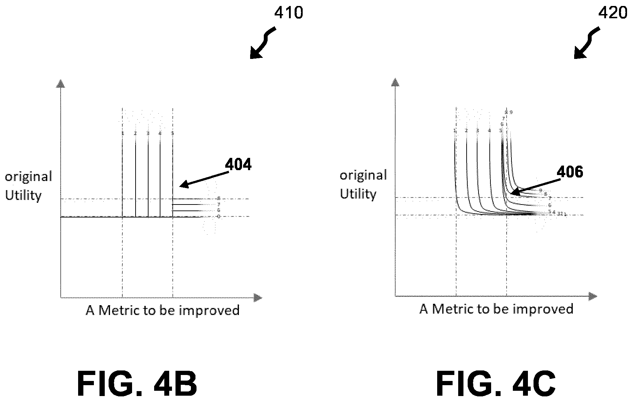

As shown in **FIGS. 4B and 4C****,** an updated utility function can be defined as a function that is based on the original utility function and the selected metric, and the shape of the function can be represented such that any treatment plan, with the original utility being less than the accepted reduction to the original utility of the current solution, gets a score that is lower than what was the new utility of the current plan (guaranteeing that the current solution is preferred also in the new optimization over any plan with unacceptable reduction in the original utility). As long as the original utility is above the lower limit, any plan that has better value in the selected metric is scoring better than a plan with a lower value in that metric. Optionally, as long as the utility remains above the lower limit, any plan that has a better value in the selected metric scores better than a plan with a lower value in that metric. Until the desired, pre-defined, level of the metric is reached, after when the new utility is again scoring plans based on the original utility. The new solution can be located in a rectangular region **404** where the new solution (e.g., a second treatment plan) which is searched. Optionally, the new solution may be located in a rectangular region **404** within which the new solution, e.g. a second treatment plan, is searched.

The updated utility function can be defined as a function of the original utility function and the metric that the clinician determines should be improved. As shown in **FIG. 4B****,** a new utility function is presented as a contour plot. The scale of the new utility function can be in accordance with one or more different scales. As shown in **FIG. 4C****,** a smooth alternative to the utility function of **FIG. 4B** is presented as a graph. Some characteristics of the utility function of **FIG. 4B** include: (1) as long as the utility value associated with a utility function stays within an accepted reduction zone (*see* **FIG. 4A**), a small reduction in the original utility does not cause a substantial difference to the new utility values, but once an original utility value starts to reach a limit of the accepted reduction, the change in the new utility is much steeper (e.g., is associated with a higher derivative); and (2) small changes in the selected metric can cause significant improvement to the new utility until the desired level is reached, at which point (3) the change in original utility has a bigger impact to the new utility.

In some embodiments, one or more of the utility functions described herein may not correlate well with gradient-based optimizers, and the utility functions can be converted to a smoother function, as presented in the **FIG. 4C****.** The function represented by **FIG. 4C** can be similar to (e.g., be associated with similar characteristics as) the utility functions of **FIGS. 4A** and **4B****,** but can also support a smooth gradient evaluation which in turn will, for every candidate solution, indicate how much one should emphasize improvement in the original utility or in the metric value when a new solution is searched in the neighborhood of the current candidate solution. The new solution can be located in a rectangular region **406** where the new solution (e.g., a second treatment plan) which is searched.

In some embodiments, the function represented in **FIG. 4C** can be approximated with a pre-defined continuous piece-wise linear function based on triangularization of the U₀, M-plane. In some embodiments, combinations of the values of *u*₀*, Δu, *m*,* Δm can be handled by performing translation and scaling operations to this pre-defined function.

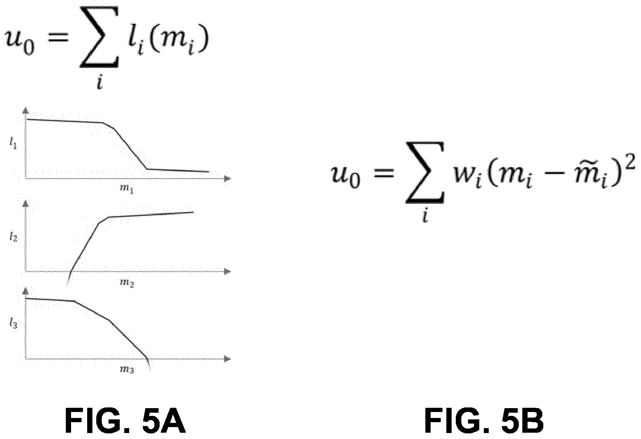

Referring now to **FIGS. 5A** and **5B** illustrated are example functions, according to embodiments. As illustrated by **FIG. 5A****,** a utility value *u*₀ can be represented as a sum of the value of argument functions *lᵢ*. In some embodiments, for a given treatment plan, the utility value *u*₀ can include a sum of the metric values for a given argument function. As illustrated by **FIG. 5B****,** a utility value *u*₀ can be represented as a weighted sum of quadratic functions. In this example, *m̃ᵢ* can include user-defined values or ranges of values (goal values) for a given metric *mᵢ*, and *wᵢ* can include user-defined weights.

In a non-limiting example, and with continued reference to **FIG. 5A****,** a first metric *m₁* can represent a metric including a dose delivered to a PTV of a patient; a second metric *m₂* can represent a dose delivered to an OAR; and *m₃* can represent conformance of delivery of energy to the PTV. In this illustrative example, an analytics server (as described herein) can determine the utility value *u*₀ based on the analytics server summing the values of the argument functions *lᵢ*. While certain metrics are described throughout, it will be understood that these metrics are examples and that utility values can be determined in accordance with different metrics or sets of metrics than those described herein. Additionally, or alternatively, the utility functions described herein can include functions that include a direct indication of a dose distribution to be delivered to the PTV of the patient.

The various illustrative logical blocks, modules, circuits, and algorithm steps described in connection with the embodiments disclosed herein can be implemented as electronic hardware, computer software, or combinations of both. To clearly illustrate this interchangeability of hardware and software, various illustrative components, blocks, modules, circuits, and steps have been described above generally in terms of their functionality. Whether such functionality is implemented as hardware or software depends upon the particular application and design constraints imposed on the overall system. Skilled artisans can implement the described functionality in varying ways for each particular application, but such implementation decisions should not be interpreted as causing a departure from the scope of this disclosure or the claims.

Embodiments implemented in computer software can be implemented in software, firmware, middleware, microcode, hardware description languages, or any combination thereof. A code segment or machine-executable instructions can represent a procedure, a function, a subprogram, a program, a routine, a subroutine, a module, a software package, a class, or any combination of instructions, data structures, or program statements. A code segment can be coupled to another code segment or a hardware circuit by passing and/or receiving information, data, arguments, parameters, or memory contents. Information, arguments, parameters, data, etc., can be passed, forwarded, or transmitted via any suitable means including memory sharing, message passing, token passing, network transmission, etc.

The actual software code or specialized control hardware used to implement these systems and methods is not limiting of the claimed features or this disclosure. Thus, the operation and behavior of the systems and methods were described without reference to the specific software code being understood that software and control hardware can be designed to implement the systems and methods based on the description herein.

When implemented in software, the functions can be stored as one or more instructions or code on a non-transitory computer-readable or processor-readable storage medium. The steps of a method or algorithm disclosed herein can be embodied in a processor-executable software module, which can reside on a computer-readable or processor-readable storage medium. A non-transitory computer-readable or processor-readable media includes both computer storage media and tangible storage media that facilitate transfer of a computer program from one place to another. A non-transitory processor-readable storage media can be any available media that can be accessed by a computer. By way of example, and not limitation, such non-transitory processor-readable media can comprise RAM, ROM, EEPROM, CD-ROM or other optical disk storage, magnetic disk storage or other magnetic storage devices, or any other tangible storage medium that can be used to store desired program code in the form of instructions or data structures and that can be accessed by a computer or processor. Disk and disc, as used herein, include compact disc (CD), laser disc, optical disc, digital versatile disc (DVD), floppy disk, and Blu-ray disc where disks usually reproduce data magnetically, while discs reproduce data optically with lasers. Combinations of the above should also be included within the scope of computer-readable media. Additionally, the operations of a method or algorithm can reside as one or any combination or set of codes and/or instructions on a non-transitory processor-readable medium and/or computer-readable medium, which can be incorporated into a computer program product.

The preceding description of the disclosed embodiments is provided to enable any person skilled in the art to make or use the embodiments described herein and variations thereof. Various modifications to these embodiments will be readily apparent to those skilled in the art, and the principles defined herein can be applied to other embodiments without departing from the scope of the subject matter disclosed herein. Thus, the present disclosure is not intended to be limited to the embodiments shown herein but is to be accorded the widest scope consistent with the following claims and the principles and novel features disclosed herein.

While various aspects and embodiments have been disclosed, other aspects and embodiments are contemplated. The various aspects and embodiments disclosed are for purposes of illustration and are not intended to be limiting, with the true scope being indicated by the following claims.

## Claims

1. A system comprising:
one or more processors configured to:
determine a first treatment plan from among a plurality of treatment plans that satisfies an initial utility value, the initial utility value determined to indicate that the first treatment plan is optimized when compared to other treatment plans of the plurality of treatment plans based on a first utility function,
where a utility value of each treatment plan of the plurality of treatment plans is represented using a first metric value associated with a first metric and a second metric value associated with a second metric for each treatment plan of the plurality of treatment plans,
receive data associated with a request to determine a second treatment plan from among the plurality of treatment plans, the request specifying a desired first metric value that is different from the first metric value of the first treatment plan;
determine the second treatment plan from among the plurality of treatment plans, where the first metric value of the second treatment plan satisfies a first metric threshold when compared to first metric values of other treatment plans of the plurality of treatment plans; and
provide data associated with the second treatment plan to cause a device to operate in accordance with the second treatment plan.

2. The system of claim 1, wherein the one or more processors that receive the data associated with the request are configured to receive the data associated with the request, the request specifying the desired first metric value and a desired utility value, and
wherein the one or more processors that determine the second treatment plan from among the plurality of treatment plans are configured to determine the second treatment plan from among the plurality of treatment plans based on the first metric value of the second treatment plan satisfying the first metric threshold and the utility value of the second treatment plan satisfying a utility value threshold.

3. The system of claim 1 or claim 2, wherein the one or more processors that determine the first treatment plan from among the plurality of treatment plans that satisfies the initial utility value are configured to:
determine the first treatment plan from among the plurality of treatment plans based on the first utility function, the first utility function representing a first set of target metrics.

4. The system of any preceding claim, further comprising:
determining an updated first utility function based on the first utility function, the desired first metric value and the desired utility value specified by the request,
wherein the one or more processors that determine the second treatment plan from among the plurality of treatment plans are configured to:
determining the second treatment plan based on an updated utility function, the updated utility function representing a second set of target metrics.

5. The system of claim 4, wherein the first utility function represents a first search space, and wherein the updated utility function represents a second search space that is at least in part different from the first search space;
and optionally:
wherein the first search space is bound by a first Pareto surface, and wherein the second search space is bound by a second Pareto surface.

6. The system of any preceding claim, wherein the one or more processors that determine the second treatment plan from among the plurality of treatment plans are configured to:
determine the second treatment plan from among the plurality of treatment plans, where the second treatment plan has a utility value that is lower than the utility value of the first treatment plan;
and/or: wherein the one or more processors are configured to:
receive data associated with a planning target volume (PTV) of a patient,
wherein the one or more processors that determine the first treatment plan from among the plurality of treatment plans that satisfies an initial utility value are configured to:
determine the first treatment plan from among the plurality of treatment plans, where the plurality of treatment plans represent operation of a linear accelerator (LINAC) delivering energy to the PTV of the patient.

7. The system of any preceding claim, wherein the first metric and the second metric each represent one of: a target coverage of the PTV, a mean dose of energy delivered to the PTV, a maximum dose for an organ at risk (OAR), a mean dose of energy delivered to the OAR, or a complexity of a treatment plan.

8. A method comprising:
determining, by at least one processor, a first treatment plan from among a plurality of treatment plans that satisfies an initial utility value, the initial utility value determined to indicate that the first treatment plan is optimized when compared to other treatment plans of the plurality of treatment plans based on a first utility function,
where a utility value of each treatment plan of the plurality of treatment plans is based on a first metric value associated with a first metric and a second metric value associated with a second metric for each treatment plan of the plurality of treatment plans,
receiving, by the at least one processor, data associated with a request to determine a second treatment plan from among the plurality of treatment plans, the request specifying a desired first metric value that is different from the first metric value of the first treatment plan; and
determining, by the at least one processor, the second treatment plan from among the plurality of treatment plans, where the first metric value of the second treatment plan satisfies a first metric threshold when compared to first metric values of other treatment plans of the plurality of treatment plans.

9. The method of claim 8, wherein the request specifies the desired first metric value and a desired utility value, and
wherein determining the second treatment plan from among the plurality of treatment plans comprises determining the second treatment plan from among the plurality of treatment plans based on the first metric value of the second treatment plan satisfying the first metric threshold and the utility value of the second treatment plan satisfying a utility value threshold.

10. The method of claim 8 or claim 9, wherein determining, by the at least one processor, the first treatment plan from among the plurality of treatment plans that satisfies the initial utility value comprises determining, by the at least one processor, the first treatment plan from among the plurality of treatment plans based on a first utility function, the first utility function representing a first set of target metrics;
and optionally: wherein the method further comprises:
determining, by the at least one processor, an updated first utility function based on the first utility function, the desired first metric value and the desired utility value specified by the request,
wherein determining the second treatment plan from among the plurality of treatment plans comprises:
determining, by the at least one processor, the second treatment plan based on an updated utility function, the updated utility function representing a second set of target metrics.

11. The method of claim 10, wherein the first utility function represents a first search space, and wherein the updated utility function represents a second search space that is at least in part different from the first search space;
and optionally: wherein the first search space is bound by a first Pareto surface, and wherein the second search space is bound by a second Pareto surface.

12. The method of any of claim 8 to claim 11, wherein determining, by the at least one processor, the second treatment plan from among the plurality of treatment plans comprises:
determining, by the at least one processor, the second treatment plan from among the plurality of treatment plans, where the second treatment plan has a utility value that is lower than the utility value of the first treatment plan;
and/or: wherein the method further comprises:
receiving, by the at least one processor, data associated with a planning target volume (PTV) of a patient,
wherein determining, by the at least one processor, the first treatment plan from among the plurality of treatment plans that satisfies an initial utility value comprises:
determining, by the at least one processor, the first treatment plan from among the plurality of treatment plans, where the plurality of treatment plans represent operation of a linear accelerator (LINAC) delivering energy to the PTV of the patient.

13. The method of any of claim 8 to claim 12, wherein the first metric and the second metric each represent one of: a target coverage of the PTV, a mean dose of energy delivered to the PTV, a maximum dose for an organ at risk (OAR), a mean dose of energy delivered to the OAR, or a complexity of a treatment plan.

14. A non-transitory computer-readable medium storing instructions thereon that, when executed by at least one processor, causes the at least one processor to:
determine a first treatment plan from among a plurality of treatment plans that satisfies an initial utility value, the initial utility value determined to indicate that the first treatment plan is optimized when compared to other treatment plans of the plurality of treatment plans based on a first utility function,
where a utility value of each treatment plan of the plurality of treatment plans is based on a first metric value associated with a first metric and a second metric value associated with a second metric for each treatment plan of the plurality of treatment plans,
receive data associated with a request to determine a second treatment plan from among the plurality of treatment plans, the request specifying a desired first metric value that is different from the first metric value of the first treatment plan;
determine the second treatment plan from among the plurality of treatment plans, where the first metric value of the second treatment plan satisfies a first metric threshold when compared to first metric values of other treatment plans of the plurality of treatment plans; and
provide data associated with the second treatment plan to cause a device to operate in accordance with the second treatment plan.

15. The non-transitory computer-readable medium of claim 14, wherein the instructions that cause the at least one processor to receive the data associated with the request cause the at least one processor to receive the data associated with the request, the request specifying the desired first metric value and a desired utility value, and
wherein the instructions that cause the one or more processors to determine the second treatment plan from among the plurality of treatment plans cause the one or more processors to determine the second treatment plan from among the plurality of treatment plans based on the first metric value of the second treatment plan satisfying the first metric threshold and the utility value of the second treatment plan satisfying a utility value threshold.
